Europäisches Patentamt

European Patent Office

Office européen des brevets

(19)

(11) Publication number : **0 436 466 A2**

(12) # EUROPEAN PATENT APPLICATION

(21) Application number : **90811006.7**

(22) Date of filing : **19.12.90**

(51) Int. Cl.$^5$ : **A61L 2/18**

(30) Priority : **28.12.89 US 458123**

(43) Date of publication of application :
**10.07.91 Bulletin 91/28**

(84) Designated Contracting States :
**AT BE CH DE DK ES FR GB GR IT LI LU NL SE**

(71) Applicant : **CIBA-GEIGY AG**
**Klybeckstrasse 141**
**CH-4002 Basel (CH)**

(72) Inventor : **Nicolson, Paul**
**7765 Sagebrush Drive**
**Dunwoody, GA 30350 (US)**
Inventor : **Seamons, Kenneth R.**
**2789 Saddlebrook Way**
**Marietta, GA 30064 (US)**
Inventor : **Tsao, Fu-Pao**
**1361 Pinehurst Hunt**
**Lawrenceville, GA 30245 (US)**

(54) **Improved control of catalytic reaction and devices therefor.**

(57)   A method of disinfecting a hydrogen peroxide stable material by contacting said material with a hydrogen peroxide containing solution and a hydrogen peroxide decomposition means adapted to permit said material to have a cumulative (% peroxide)·(min) exposure from the time said decomposition means contacts said hydrogen peroxide containing solution over a period not exceeding 12 hours of at least 20 % (peroxide)·(min). Also, disclosed are devices and components thereof whereby said method can be achieved.

**Fig. 1** [PRIOR ART]

EP 0 436 466 A2

# IMPROVED CONTROL OF CATALYTIC REACTION AND DEVICES THEREFOR

## Field of the invention

The invention relates to hydrogen peroxide disinfection methods and devices. It is especially relevant to those methods and devices particularly adapted to the $H_2O_2$ disinfection of contact lenses, most especially soft contact lenses. Further, the invention generally relates to catalytic reactions where the reaction is to proceed over a period of time but not in a homogeneous manner. It especially relates to catalytic breakdown of hydrogen peroxide.

## Background of the invention

In the area of peroxide disinfection of surfaces, residual peroxide must be removed from the disinfected surface due to sensitivity of other materials with which the disinfected surface comes in contact. This is especially so in the contact lens disinfection area where the disinfected lens will be placed directly on the eye. In the past, this has been accomplished by methods such as dilution with large volumes of water or saline or exposing the solution and/or disinfected material to a hydrogen peroxide decomposing agent or catalyst for a time sufficient to reduce the residual hydrogen peroxide to acceptable levels.

Unfortunately, in many settings, especially in the contact lens field, dilution is not a commercially viable or user practical alternative. Furthermore, where the decomposition agent or catalyst is used, the regimen becomes bothersome in the number of steps involved and the degree of user involvement necessary. As the complexity of the regimen goes up, strict compliance with that regimen drops off dramatically. Hence, more user friendly single step processes have been attempted. Most notable and relevant to the instant invention in current use is the AO Sept system. This system operates by placing a contact lens to be disinfected in contact with a solution of peroxide and a platinum disk whereby peroxide disinfection and decomposition occur essentially simultaneously. The only user input is to place the components in the system and wait the appropriate time interval before removing the lenses.

Unfortunately, many times users do not follow the regimen sufficiently so that there is the risk that complete disinfection has not occurred or decomposition has not sufficiently taken place.

In a typical hydrogen peroxide system in which the hydrogen peroxide is contacted with a hydrogen peroxide decomposing catalyst with the simultaneous introduction of contact lenses for disinfection, the hydrogen peroxide depletes rapidly and the disinfection at the higher concentrations is short. For example, in an AO Sept system in which the initial concentration of hydrogen peroxide is 3 %, the concentration of the hydrogen peroxide falls rapidly to about 0.1 % in about 12.5 minutes. After this point, the concentration decreases very slowly and it takes several hours, i.e. up to 8 hours or more, before the hydrogen peroxide is depleted sufficiently to ocularly safe or acceptable levels whereby the contact lens can be inserted into the eyes without fear or irritation or injury.

In some instances, it is desirable, however, to control the catalytic reaction of the hydrogen peroxide such that concentration of the hydrogen peroxide is high over a longer transition period and ideally to control the system so that the hydrogen peroxide concentration can be maintained at high levels for a longer period of time and then abruptly reduced to ocularly acceptable levels. This longer transition time is especially important where the materials to be disinfected are heavily contaminated. There is, therefore, a need for an improved hydrogen peroxide disinfectant system which makes it possible to control the decomposition rate of the hydrogen peroxide.

## Objects of the invention

It is an object of the present invention to provide an improved hydrogen peroxide disinfection regimen than those currently available.

It is another object of the invention to provide a simple single-step disinfection method for use with hydrogen peroxide.

It is still another object of the invention to provide a method of carrying out a catalytic reaction in a controlled manner which allows for greater transition times for the material to be disinfected in an $H_2O_2$ solution at higher $H_2O_2$ concentrations than in prior art hydrogen peroxide-catalyst systems.

It is a further object of the invention to provide a method of carrying out a catalytic reaction in a controlled manner allowing for greater transition times of a substance in the reactant mixture prior to the catalytic reaction occurring to a significant degree in the vicinity of that substance.

It is another object of the invention to provide a contact lens hydrogen peroxide disinfection system and method which avoids the aforementioned problems and/or difficulties.

## Summary of the invention

The present invention relates to a method of disinfecting a hydrogen peroxide stable material by contacting said material with a hydrogen peroxide containing solution and a hydrogen peroxide decomposition means adapted to permit said material to have a longer exposure at higher concentrations than previously afforded by conventional peroxide disinfectant systems. This is achieved by utilizing a hydrogen peroxide decomposition means which permits the material to be disinfected such as to have a cumulative (% peroxide)·(min) exposure from the time the decomposition means contacts the hydrogen peroxide containing solution over a period not exceeding 12 hours of at least 20(% peroxide)·(min). Also disclosed are devices and components whereby said method can be achieved.

The invention also relates to a method of buoyantly controlling a catalytic reaction comprising contacting a liquid reaction medium and a particulate catalyst capable of converting said liquid reactant medium into a reaction product, said particulate catalyst having a density such that it (a) remains substantially at the reactant medium/reaction product interface ; (b) it sinks into the reactant medium but generates a positive buoyant force which adheres for a time to said particle driving said particle toward the reactant solution surface or total solution surface, the reaction product thereof being as dense or less dense than said reactant medium ; or (c) it rises into the reactant medium but generates a negative buoyant force which adheres for a time to said particle driving it toward the reaction product solution therefrom, said reaction product being more dense than said reactant solution ; and upon losing said buoyant force, attempts to return to its pre-buoyant force position until a new buoyant force is generated ; and to a device for carrying out said method.

Said device comprises a reaction vessel capable of containing a liquid reactant medium, a means for inputting said reactant medium, a means for removing a reaction product resulting from said cataytic reaction, and a catalyst particle which (a) has a buoyancy in said reactant medium/reaction product which maintains said particle substantially at said reactant medium/reaction product interface, or (b) develops a buoyant force in the course of said catalytic reaction which adheres to said particle for some time so as to drive said particle toward said reaction product until said buoyant force disengages itself from said particle at which time said catalytic particle attempts to return to its former position in said reactant medium.

## Description of the drawings

Fig. 1 : Diagram showing a peroxide concentration versus time graph of the prior art.

Fig. 2 : Diagram showing a peroxide concentration versus time graph of the invention.

Fig. 3 : Diagram demonstrating the effect of the rate of catalytic reaction (initial oxygen liberation rate) on cumulative exposure.

Fig. 4 : Sectional view of one embodiment of the invention : tube for catalyst having orifices.

Fig. 5 : Diagram showing the cumulative exposure versus time graph of the examples.

Fig. 6 : Hinged catalyst system washer, back view : catalyst in up-position.

Fig. 7 : Hinged catalyst system washer, side view : catalyst in down-position.

Fig. 8 : Diagram showing cumulative exposure versus time graph for hinged catalyst systems from zero to 30 minutes.

Fig. 9 : Diagram showing cumulative exposure versus time graph for hinged catalyst systems from zero to 360 minutes.

Fig. 10 : Sectional view of a stir bar system.

## Detailed description of the invention

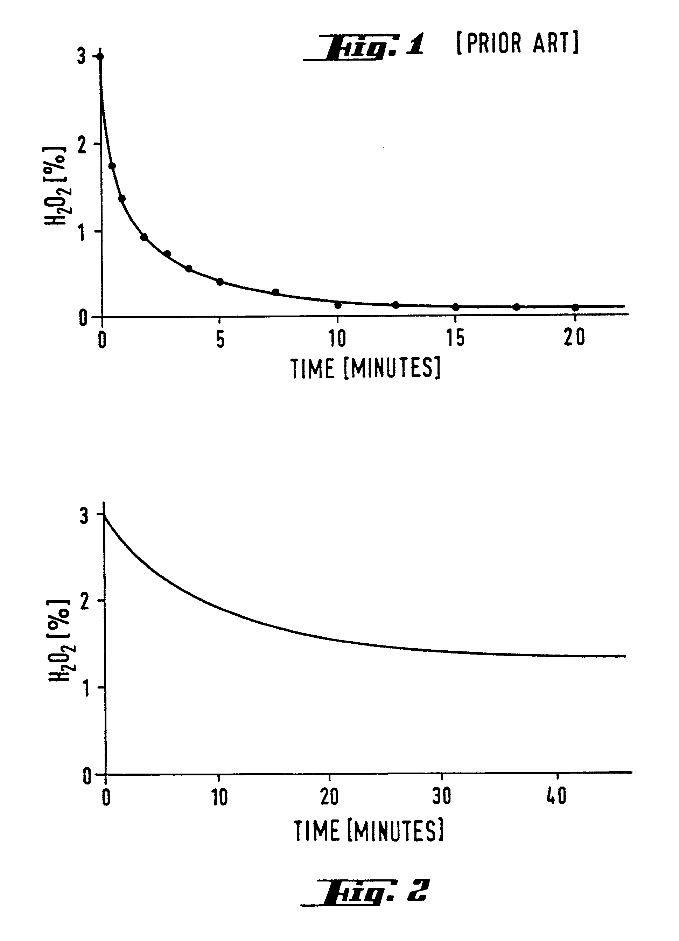

The cumulative exposure described in the present application is that exposure from the time the composition means, such as a catalyst, contacts the peroxide containing solution, i.e. time zero, over a period of no greater than 12 hours of at least 20 % peroxide·min. This cumulative exposure is a convenient mathematical means of determining the rate of decomposition of the hydrogen peroxide in the hydrogen peroxide system. It is arrived at by plotting the percentages of hydrogen peroxide against time as in Figs. 1 and 2, determining the area under the curve at various time invervals and plotting this cumulative area against the time.

In Fig. 1 a typical prior art AO Sept system is depicted in which a platinum catalyst is contacted with a 3 % hydrogen peroxide solution and the rate of decomposition plotted on the graph. In such situation, it will be noted that the concentration of $H_2O_2$ falls rapidly to about 0.1 % in 12.5 minutes.

Fig. 2, however, represents a typical decomposition profile of a hydrogen peroxide system in which the rate of decomposition of the hydrogen peroxide is controlled so that the concentration of hydrogen peroxide decreases more gradually over time.

To determine the cumulative area, the area under the curves in Figs. 1 and 2, for example, are integrated or calculated at various time intervals to determine the cumulative area or the sum of the areas in terms of percent peroxide-min. These areas in turn are plotted against time to determine the rate at which the hydrogen peroxide decomposes with time. The cumulative exposure vs. time graph is thus an accurate way of mathematically expressing the disinfectant capacity of the system at any given time.

As previously pointed out, it is desirable to control the rate of decomposition so that a relatively higher concentration of hydrogen peroxide remains in the system over a longer transition time. As can be seen by Fig. 1, the area under the curve from 0 to some positive time interval, for example 5 minutes, is smaller than the area under the curve from 0 to 5 minutes in Fig. 2. Thus, the higher the cumulative area, the lower the decomposition rate of the hydrogen peroxide. In this connection, note that the cumulative area is expressed in % (peroxide)(minutes) because the ordinate of the curve is expressed in percentages and the abscissa in minutes. The calculated value of the area is, therefore, expressed in terms of % peroxide-min.

According to the present invention, the cumulative area or cumulative exposure is calculated from such graphs as Figs. 1 and 2 above, and is plotted against time to determine the cumulative exposure at various time intervals. Such a typical graph is shown in Fig. 3 of the drawings in which the effect of the catalyst rate (of decomposing the $H_2O_2$) is depicted therein.

According to Fig. 3, the prior art AO Sept system with the platinum catalyst fixed at the bottom thereof (and which has not been modified to control the cumulative exposure as in the present invention) is a relatively straight line represented by line 1 in the graph in which the cumulative exposure is about 10. At the right hand side of the graph, there are depicted the initial oxygen liberation rates. It can be seen that for the conventional AO Sept system, a high liberation rate of oxygen of about 40 ml/min is initially generated. The oxygen liberation rate, of course, is a means of determining the decomposition rate of the hydrogen peroxide, since the hydrogen peroxide is decomposed by means of the catalyst to produce water and nascent oxygen. The oxygen liberation rate is, therefore, a means of determining the rate of decomposition and the cumulative exposure of the material to be disinfected. It can be further seen from this graph that as the cumulative exposure rises above 20 % min, the generation of the oxygen is greatly decreased to between about 15 ml/min to about 1.0 ml/min, depending on the means adopted to control the cumulative exposure of the contact lens, etc. to be disinfected. This particular graph is designed to illustrate how the cumulative exposure is related to the decomposition rate of $H_2O_2$ in the system. Lines 2, 3, 4 and 5 of Fig 3. are illustrating decomposition systems having initial oxygen liberation rates of 10 ml/min (2), 5 ml/min (3), 2 ml/min (4) or 1 ml/min (5).

There are several practical means of controlling the disinfectant capacity of the hydrogen peroxide system such that the material to be disinfected has a cumulative exposure of at least 20 % peroxide-min over a period of no greater that 12 hours. Before discussing such concrete means, a general description of the primary considerations which go into the catalytic decomposition of hydrogen peroxide will be discussed.

There are generally five important steps to consider in the catalytic decomposition of hydrogen peroxide, which steps can be broadly described as follows :

(1) the transportation of the $H_2O_2$ to the catalyst to insure a continuous contact between the catalyst and hydrogen peroxide ;

(2) the absorption of hydrogen peroxide to the catalyst surface ;

(3) the neutralization or catalysis in which the hydrogen peroxide is decomposed to water and nascent oxygen ;

(4) the desorption from the surface of the reaction products, i.e. the water and nascent oxygen, or other contaminants so as to reexpose the active sites ;

(5) the transportation of the reaction products away from the surface.

There are various concrete means to control the decomposition rate of the hydrogen peroxide by taking into consideration the factors which go into the catalytic decomposition as described above, such as to control the cumulative exposure of the material to be disinfected in the hydrogen peroxide solution.

One such means is to prepoison the catalyst before it is sold and subjected to the first use by the user. In a typical catalytic decomposition of hydrogen peroxide, the active sites of the catalyst are naturally deactivated to various degrees over a period of time by absorption or contamination of the surface with the reaction products, etc. Such deactivation, in turn, slows down the decomposition rate of the hydrogen peroxide whereby the cumulative area under the curve or cumulative exposure as described above is increased. In order to control the catalytic rate of decomposition, according to the present invention, a catalyst, such as platinum, is prepoisoned by, for example, subjecting it to a hydrogen decomposition catalytic reaction to partially deactivate the active sites prior to sale to the user of the catalyst-hydrogen peroxide decomposition device so that the cumulative exposure is controlled from the outset. To determine whether the catalyst is sufficiently prepoisoned, the generation of oxygen from the system can be measured. Thus, as previously described, in a typical AO Sept system using platinum as a catalyst, the initial generation of oxygen is 40 ml/min, see Fig. 3, right hand side of the drawings. To sufficiently prepoison the catalyst so that the cumulative exposure is over 20 %·min, the oxygen liberated during the reaction is periodically measured until the oxygen liberation rate is somewhere between 15 ml/min and preferably between about 2 and 5 ml/min to correlate with the minimum 20 % minimal exposure shown on the graph.

In another means of controlling the cumulative exposure, the catalyst can be partially enclosed in a container such as a tube with openings in the tube to control the escape of oxygen from the system to approximately 2-15 and preferably 2-3 ml/min. This device can be a simple tubing containing one or more small orifices at the top or bottom calibrated to leak out the oxygen to an amount approximating 2-15 ml/min and preferably 2-3 ml/min. Such a device is described in Fig. 4 of the drawings. The tubing (6) comprising a catalyst (7) has small orifices (8) at the top. The device or tubing can be further modified such that the sides of the tube contain tiny perforations therein to permit the escape of the oxygen gas through said perforations. Althougs a tube is depicted therein, a container of any shape can be employed as long as it is calibrated to control the escape of oxygen from the catalyst.

Another convenient means of controlling the decomposition of the hydrogen peroxide and the cumulative area under the curve as described above is through a buoyancy mediated control of the catalytic reaction as will be subsequently described in detail.

The catalyst used also is an important factor in controlling the decomposition rate. The activity of various catalysts differs considerably with such catalyst as platinum (Pt) exhibiting a high decomposition rate per unit surface to gold (Au) which has a decomposition rate which may be practically too low, for most $H_2O_2$ disinfectant systems. The order of $H_2O_2$ decomposition/unit surface area for various catalyst systems is as follows :

$$Pt > Os > In > Pd > Ru > Rh > Au.$$

The selection of the proper catalyst with any of the aforementioned means is therefore an important factor. Although any of such catalyst, among others conventionally employed, may be used to decompose $H_2O_2$, it is preferred to use a catalyst having a high rate of decomposition, such as Pt, and to modify it to slow down the reaction rate according to one of the procedures outlined above.

As a specific procedure of controlling the decomposition rate of the $H_2O_2$ and the cumulative exposure, the buoyancy mediated catalytic control embodiment of the present invention will be described in detail as a means of exemplifying the characteristic features of the present invention.

Buoyancy mediated control system for increasing the exposure time at higher concentrations

According to such buoyance mediated control system, the catalytic reaction is carried out in (a) a $H_2O_2$ liquid which generates a gas (oxygen) that gives the catalytic particle sufficient buoyancy to rise to the surface of the liquid phase or (b) in a liquid wherein the reaction product solution is of a significantly different density than and substantially non-miscible with the reactant solution and the catalytic particle is of appropriate density that at least a portion thereof tends to remain in contact with the reactant solution portion and of sufficient buoyancy so as to be at or near the reactant solution/reaction product solution interface. The present invention is primarily interested in the first reaction (a).

Buoancy controlled catalytic reactions fall into two primary types of reactions. First are those reactions which generate a gas. The gas bubbles adhere to the surface of the catalyst particle creating a buoyant particle. The buoyant particle rises to the surface where the gas bubble escapes to the gas phase over the liquid reaction medium. Upon losing the gas bubbles, the catalyst loses buoyancy and begins to descend until it again contacts liquid containing reactants so that further buoyant gas bubbles can be generated. This bobbing action, is therefore confined to the uppermost layers of the reactant solution and the reaction product solution, leaving the lower portion of the reactant solution substantially undisturbed for a significant portion of time.

Depending on the speed with which a particular reaction is intended to take place, the buoyancy of the catalyst can be altered by coating a carrier or substrate particle of particular density with varying amounts of catalyst. The less dense the particle, the more it will extend the reaction time as such a particle will be confined more to the uppermost reactant solution portions and reaction product solution. Similarly, the greater the amount of catalyst at the particle surface, the more the particle will find itself in the uppermost reactant solution and reaction product solution. Particle shape also plays a part in reaction rate control. Spherical particles will tend to lose their gas bubble lifters quicker than other shapes, yet they move more easily through the liquid solutions than other particle shapes. Finally, particle size is important in control of the reaction rate in relation to changes in particle weight versus particle surface area. The greater the weight, the more buoyant force needed from gas bubbles ; if surface area does not increase at least as rapidly, then the particle will reside for a greater time in the reactant solution area and thereby speed up the reaction more so than otherwise. Counterbalancing this is the fact that the greater the catalyst surface area, the faster the reaction, and the shorter the residence time of the article to be disinfected in the disinfectant.

In the second type of buoyancy controlled catalytic reaction, the catalytic particle resides, due to its density, at or near the reactant solution/reaction product solution interface, at least part of it tending to remain in contact with the reactant solution. If the reaction product solution is less dense than the reactant solution, then the reaction proceeds substantially from top to bottom and the catalytic particles are designed to be slightly less dense than the reactant solution (i.e. between the reaction product and reactant solution densities). If the reaction product solution is more dense than the reactant solution, then the reaction proceeds from bottom to top and the catalytic particle is designed to be slightly more dense than the reactant solution. In either event, the catalytic particle must return to contact reactant solution if the reaction is to continue to proceed. If the reaction product adheres to the catalyst for sufficient time to drive the catalyst toward the reaction product solution, the catalyst particles may also be of the same density as the reactant solution, in either of the cases described.

Typical catalytic reactions of the first (i.e. gas) type include, but are not limited to, hydrogen peroxide solution with peroxidase, catalase, or transition metals such as platinum, palladium, etc. and compounds thereof such as iron oxide, manganese oxide or titanium oxide ($TiO_2$) catalyst particles. In fact, such catalyst systems can be employed in all of the decomposition means contemplated by the present invention.

Typical catalytic particles can be prepared from refractory, plastics, fiber, etc. particles having an appropriate configuration which are then overcoated or impregnated with catalyst, such as platinum, catalase, etc.

Convenient particle shapes include but are not limited to discs, plates, spheres, stars, "donuts", wafers, tubes, rods and strips. Essentially any particle shape will suffice.

Suitable particle sizes for spheres are particles with diameters of from about 0.5 mm up to about 5 cm, preferably about 1 mm to about 2 cm, more preferably about 3 mm to about 1 cm, most preferably about 4 mm to about 8 mm. Suitable, although not limiting for a plate or disc, or wafer shape is 1 cm x 2 cm x 0.02 cm. Appropriate size limits for other shapes can readily be inferred by those of ordinary skill. To balance particle size and surface area to the desired ratio, one may use multiple smaller size particles, for example 3 particles of 1 mm diameter each instead of one particle of equivalent weight or surface area.

For the second embodiment of the buoyancy mediated control system, the catalyst particle does not vary far from the reactant solution/reaction product solution interface, and particle movement considerations are less important. Hence, there is greater flexibility in terms of catalyst shape and more concern with appropriate density for the particle. While it is expected that there is going to be little if any "bobbing" action, such movement is not precluded. In fact, to the extent the reaction product generated by the catalytic reaction adheres to the catalyst, there may be some significant positive or negative buoyant effect (depending upon whether the product solution is less dense or more dense than the reactant solution). Beyond these concerns, the catalytic particles for use in the first embodiment are also suitable for use in the second embodiment of the present invention.

The gas producing reaction contemplated herein represents the degeneration of hydrogen peroxide to water and oxygen gas. In these gas generating reactions, preferable particle shapes are stars, plates, discs, wafers, spheres, rods, strips and donuts ; more preferably, spheres, donuts, rods, plates, discs, and wafers ; most preferably plates, discs, wafers, and spheres.

The particular density of the catalytic particle is dependent upon how fast and far the particle is to penetrate back into the reactant solution phase. For a given shape, the greater the density, the longer it will take to generate sufficient buoyant force to lift it out of the reactant solution phase (in gas generating systems and those where reaction products are less dense than reactants), yet as the surface area increases relative to weight, the less time the particle will spend in the reactant solution.

For the hydrogen peroxide/oxygen gas system of this invention, the preferred parameters are such that the density of the catalyst particle is from 1.05 to about 1.30, preferably from about 1.10 to about 1.20, most preferably from about 1.12 to about 1.18 ; the surface area of the particle of about 0.008 to about 75 cm$^2$, preferably about 0.03 cm$^2$ to about 13 cm$^2$, more preferably about 0.2 cm$^2$ to about 5 cm$^2$, most preferably about 0.3 cm$^2$ to about 3.2 cm$^2$ ; and surface area/weight ratio of the particle of from about 0.85 to about 116, preferably about 1.15 to about 54.5, most preferably about 2.95 to about 11.3.

The surface may be anything from smooth to rough with rough surfaces provided a larger effective surface area than the same sized and shaped particle of smooth morphology. The rough morphology will also allow for greater "lifter" adherence.

The hydrogen peroxide/oxygen gas system has a decomposition profile, such that total area under the % peroxide (in the vicinity of the lens) vs. time is in excess of at least 20 %·min, preferably 47 %·min, more preferably in excess of 50 %·min, even more preferably greater than about 75 %·min, still more preferably more than about 100 %·min, most preferably in excess of about 120 %·min, and ideally more than about 140 %·min, especially when using a current commercially available AO Sept cup, approximately 3 % peroxide, and sampling the peroxide in the vicinity of the lens with the lens placed standing on edge at the bottom of the cup and the cup filled to the indicated line. A commercially available AO Sept cup has been used as a sample cup in these tests for conformity with the decomposition profile.

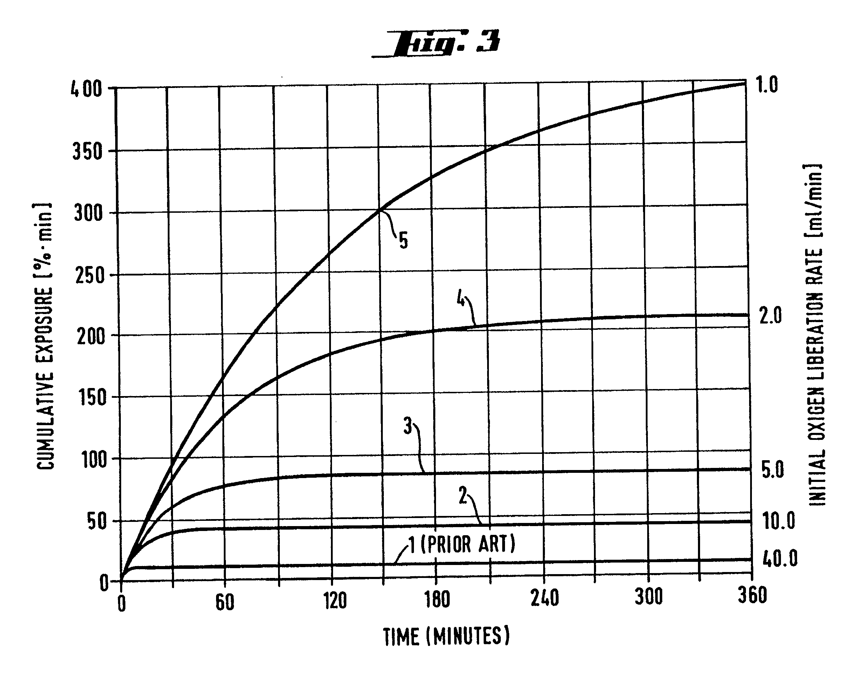

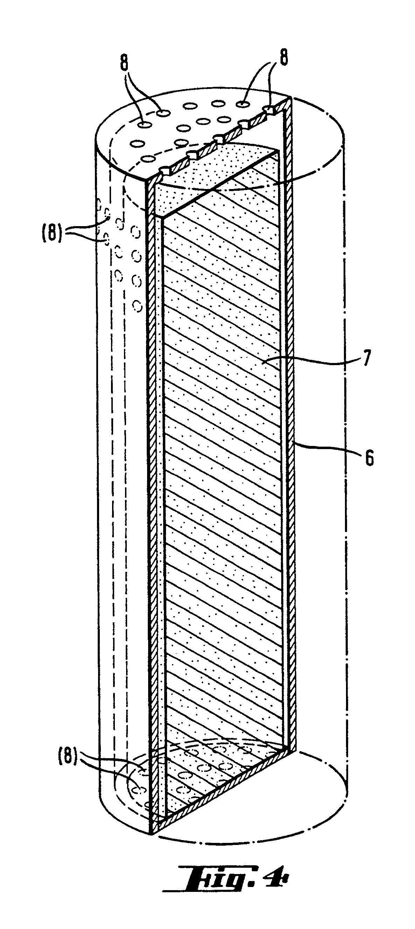

The cumulative area under the peroxide % vs. time curve is shown in Fig. 5 for three examples, which will subsequently be described.

Furthermore, in the hydrogen peroxide/oxygen gas system the residual peroxide content should be with ocularly tolerable levels preferably within less than about 8 hours, preferably less than about 6 hours, more preferably less than about 4 hours, still more preferably less than about 2 hours, most preferably less than about 90 minutes.

Still further the peroxide content should be, at the sample point, at or in excess of a disinfectantly effective concentration (i.e. at or more than 1 %) for at least 15 minutes, preferably at least 20 minutes, more preferably at least 30 minutes after decomposition of the approximately 3 % hydrogen peroxide solution has begun. When starting with a greater concentration of peroxide, this time period can be shortened provided the % time value for the time the peroxide concentration exceeds 35, preferably 40, in the first 15 minutes of decomposition and exceeds 40, preferably 50, more preferably 55 in the first 20 minutes of decomposition and exceeds 45, preferably 50, more preferably 60, most preferably 80, in the first 30 minutes of decomposition.

In the embodiment of the buoyancy mediated control system as described above, similar considerations apply with appropriate concern for whether the reactant solution is more or less dense than the reaction product solution.

The carrier or substrate for the catalyst can be any material that is resistant to the chemical activity of hydrogen peroxide, the catalyst being used, and oxygen gas. Most preferable for the substrate is a synthetic plastic. Of these, the material of choice is a polycarbonate, especially polycarbonate mixed with acrylate butadiene styrene.

The substrate can be prepared in its desired size and shape by well known techniques, including : extrusion, molding, cutting, chipping, milling, lathing, and/or grinding, and coated with catalyst by spraying, dipping, fluidized bed techniques, vapor phase deposition, or any other suitable known coating technique so long as the catalyst is not destroyed in the process.

The present invention will be more fully understood with reference to the following examples which illustrate one means of controlling the cumulative exposure of a contact lens to be disinfected by means of a buoyancy mediated control of a catalytic reaction. These examples are in no way to be understood as limiting the scope of the invention.

Test procedures are as follows :

Commercial AOSept® cups are filled to the pre-marked line with about 3.3 % hydrogen peroxide (about 9 ml). The system, for peroxide determination, is left open, without the cap or stem inserted. A 50 µl sample is taken and assayed. The catalytic particles used for the invention are placed into the container. Additional 50 µl samples are withdrawn at the specified times from the bottom of the cup (approximately 22 mm from the upper surface of the solution).

Example 1 : 6 of 6 mm diameter (pre-coating size) platinum coated balls (polycarbonate mixed with acrylate butadiene styrene, pre-coating density is 1.11 g/ml) are put in AOSept® cups, filled with AOSept® solution (3-3.5 % $H_2O_2$ with phosphate buffered saline) up to the line, and the concentration of $H_2O_2$ 23-25 mm from the upper surface is measured during the interval time. The results are listed on the following table. The (% peroxide times minutes) versus minutes curve is shown in line (11) of Figure 5.

Example 2 : The procedure of example 1 is repeated except that 4 of 8 mm diameter (pre-coating size) balls (Noryl, pre-coating density is 1.06 g/ml) are employed. The results are listed on the following table. The (% peroxide times minutes) versus minutes curve is shown in line (12) of Figure 5.

Example 3 : The procedure of example 1 is repeated except that 6 of 4 mm diameter (pre-coating size) balls (polycarbonate, pre-coating density is 1.19 g/ml) are employed. The results are listed on the following table. The (% peroxide times minutes) versus minutes curve is shown in line (13) of Figure 5.

Table relating to examples 1 to 3 :

What is listed for examples 1 to 3 is the concentration of hydrogen peroxide (%) at the time of interval (minutes) and the concentration of hydrogen peroxide (ppm) after 6 hours.

| Time (min) | Examples | | |
|---|---|---|---|
| | 1 | 2 | 3 |
| 0 | 3.3 (%) | 3.3 (%) | 3.3 (%) |
| 5 | 3.0 | 3.1 | 3.1 |
| 7 | 3.1 | 3.1 | 3.0 |
| 10 | 3.1 | 3.2 | 2.9 |
| ·15 | 3.0 | 2.8 | 1.8 |
| 20 | 2.7 | 2.6 | 1.1 |
| 25 | 3.0 | 2.4 | 0.4 |
| 30 | 2.9 | 2.5 | 0.3 |
| 40 | 2.8 | 2.5 | 0.2 |
| 50 | 2.3 | 1.6 | 0.0 |
| 60 | 2.0 | 1.5 | - |
| 75 | 1.7 | 1.2 | - |
| 90 | 0.3 | 0.2 | - |
| 105 | 0.0 | 0.0 | - |
| 120 | - | - | - |
| 6 (hrs) | 60.61 (ppm) | 4.5 (ppm) | 2.3 (ppm) |

The previous examples exemplify one means of controlling the cumulative exposure rate to at least 20 %·min by means of a buoyancy mediated control system. There are other ways of controlling the buoyancy mediated catalytic reaction by flexibly attaching the catalyst to the container in which the hydrogen peroxide decomposition is carried out. This can be done by attaching it to the wall of the container by means of a hinge or a flexible plastic material. This embodiment will be described in respect to the case where a hinge is used, however, it should be understood that other means can be employed to attach the catalyst to the $H_2O_2$ decomposition devices.

Hinged catalyst decomposition mean

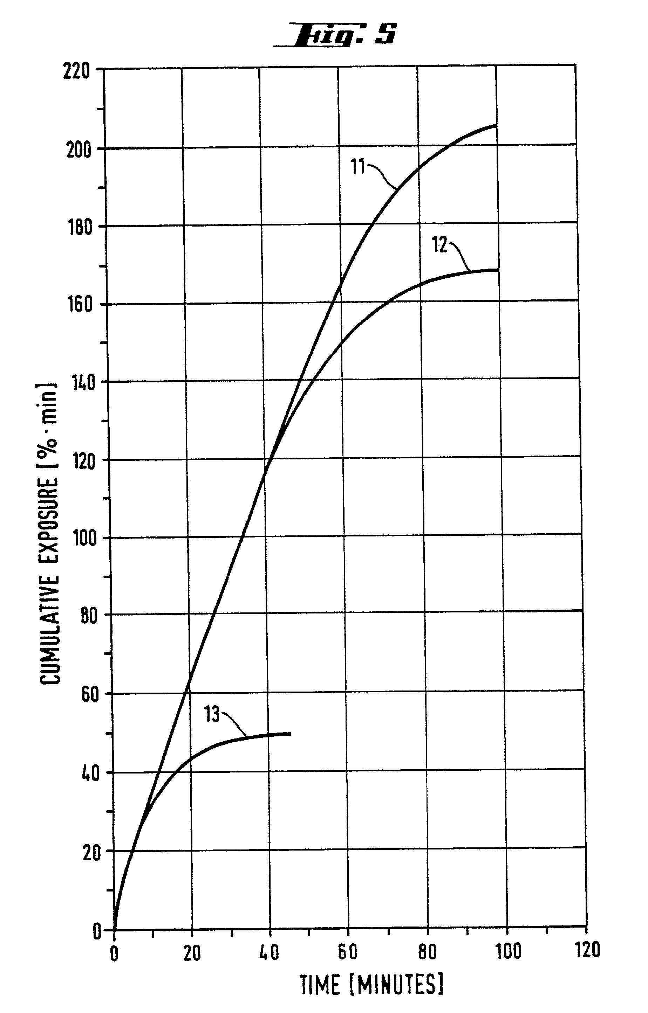

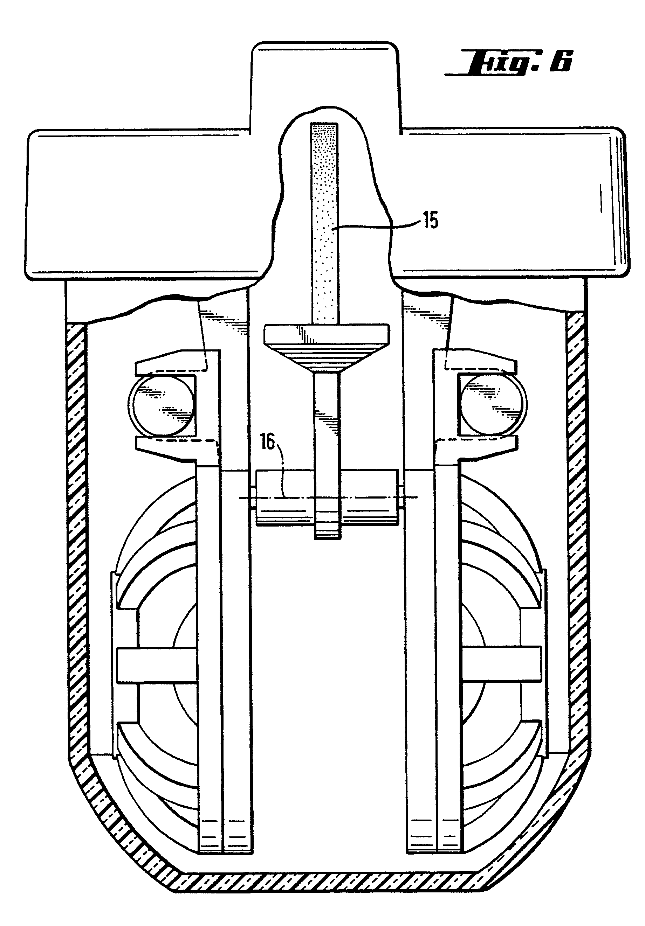

One variation of the buoyancy mediated control of the catalytic reaction resides in attaching the catalyst, such as platinum, by means of a hinge to a device in which the catalyst can be lowered and raised in the reaction solution by pivoting around a hinge as shown in Figs. 6 and 7 which respectively describe a back view of the catalyst in the up position (Fig. 6), and a side view of the catalyst in the down position (Fig. 7). In these two figures, conventional elements are not specifically mentioned since these are known to the man skilled in the art. The relevant elements are the catalyst support (15) being movably mounted via the hinge (16).

According to this embodiment, the average density of the catalytic part must be greater than the density of the solution, otherwise, it will not sink. It must be sufficiently greater such that its sinking will occur over a

8

reasonably short time after the lenses have been adequately disinfected. On the other hand, the density of the catalyst must be sufficiently close to the density of the hydrogen peroxide reaction solution, such that the interreaction with the liberated oxygen is adequate to float the catalyst into its "up position" reasonably quickly when first installed in the solution.

According to the preferred aspects of this hinge shaped device, the most common physical shape of the catalyst used is a flat plate which is horizontal in the up position and which is as wide as permitted by the clearance between the lens holders. The bottom of the plate is often slanted upward, similar to a ship's keel and a horizontal plate attached to the hinge by an uncoated arm. The configuration, as shown in the drawing, generates good front and end performance (about 1 % at 60 min), but typically has a high residual peroxide (75-150 ppm) at 6 hours. The size of the embodiment shown in the drawing is approximately 8 mm wide by 20 mm long up to about 3 mm deep.

A vertical fin can be added to increase the surface area and surface area distribution of the catalyst. The fin is intended to rise above the solution surface when in the up position where it does not speed up the front-end decomposition profile. To raise this fin out of solution, it requires a much greater lifting moment. The larger moment decreases the chance of the catalyst from "hanging up" in the up position. The catalyst typically has a clearance as large as possible, i.e. 1-2 mm between the cup wall during transition from its up to down position. Variation of this device can be used in which 2, 3 or more side fins can be used. Also, vertical fins or side fins perforated to decrease the surface area may be used.

In addition to the above, the mass distribution of the catalyst should be such that the mechanical moment about the hinge axis is less when the catalyst is in the up position.

This is accomplished by having the center of the mass of the catalyst just forward of a vertical line through the hinge access, but well above the axis. On the other hand, the horizontal distance of the vertical projection of the center of the mass from the axis is less when the catalyst is in the up position than when it is down. This causes the rotational moment to increase as the catalyst moves from the up to the down position. Therefore, less buoyancy is required in this instance to maintain the catalyst in the up position. This permits it to remain in the up position longer. Also, longer float time yield higher exposures to the disinfectant and improve efficiency. When the catalyst begins to sink, according to this configuration, it will sink quicker due to the increasing rotational moment.

The center of gravity for the catalyst produced can be modified as pointed out above by drilling out holes in the fins, cutting out slots, etc., in an effort to develop a catalyst with good lifting and sinking performance. Further, the catalyst should be near the center of the system. The internal cup diameter should be as narrow as possible in order to pack the solution around the catalyst. The inside diameter of the cups holding the hinge catalyst are about 24 - 25 mm. The bottom of the cup is rounded for the same reason. The current AO cup is 22 mm in diameter.

The amount of surface in contact or intimate contact with the solution is such that the neutralization rate can be reduced during the initial phase by limiting or preventing contact between the solution and the catalyst. This will increase the percent-min exposure to the disinfectant. The hinge catalyst lifts a segment of the catalyst out of the solution which reduces the total activity. A similar effect can be achieved by reducing the exchange rate of the lower concentration near the catalyst with higher concentration solution away from the catalyst which can be achieved by blocking structures around the catalyst, control of bubble adherence, trapping gas around the catalyst, or by control of the circulation pattern.

The wettability of the catalyst by the solution/bubble adherence is important. That is, the manner in which the liberated oxygen bubbles from the hydrogen peroxide reaction nucleates, expands and releases from the catalyst surface determines how much buoyancy the bubbles impart to the catalyst. Bubbles will become larger on a less wettable surface before they break. Larger bubbles will typically impart more buoyancy per unit of catalyst surface area, but the buoyancy of the catalyst also tends to be less responsive to the concentration of the peroxide. The reverse situation exists for smaller bubbles. The more catalyst surface, the smaller the bubbles will be when they are released.

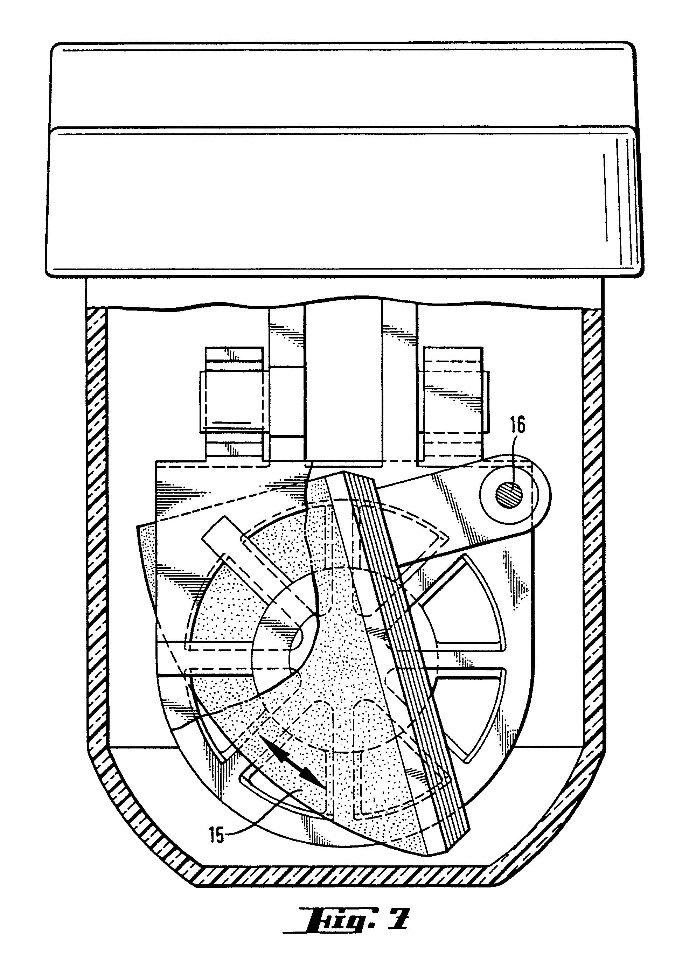

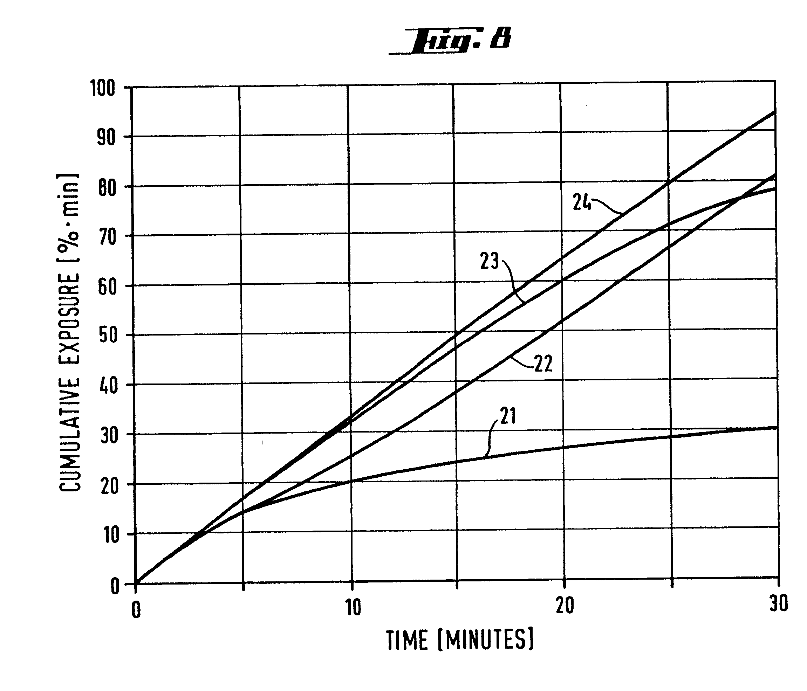

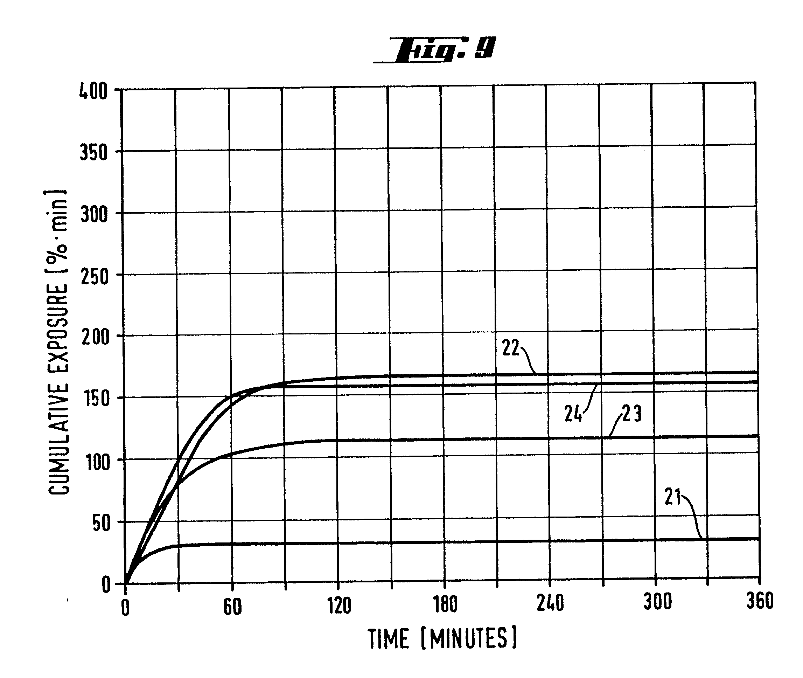

The wettability of the catalyst surface can be increased by "plasma treatment" by alloying the platinum catalyst with gold or palladium from the sputtered target. In addition, the wettability of the catalyst can be modified by the selection of materials or method of manufacturer's surface finish or by modification of the solution with a wetting agent such as propylene glycol, glycerol, or pluronic L-31. The cumulative exposure of a platinum catalyst in a 3 to 3.5 percent hydrogen peroxide solution is illustrated in Figs. 8 and 9 in which the cumulative exposure of the hinged catalyst systems are plotted against various time intervals varying from up to 30 minutes to up to about 6 hours. In such graphs, there are four lines (21), (22), (23) and (24) which are based upon platinum catalyst systems of different cumulative exposure rates, based upon the wettabilities of the catalysts, etc., which fall within the cumulative exposure parameters of the present invention.

### Stir bar system

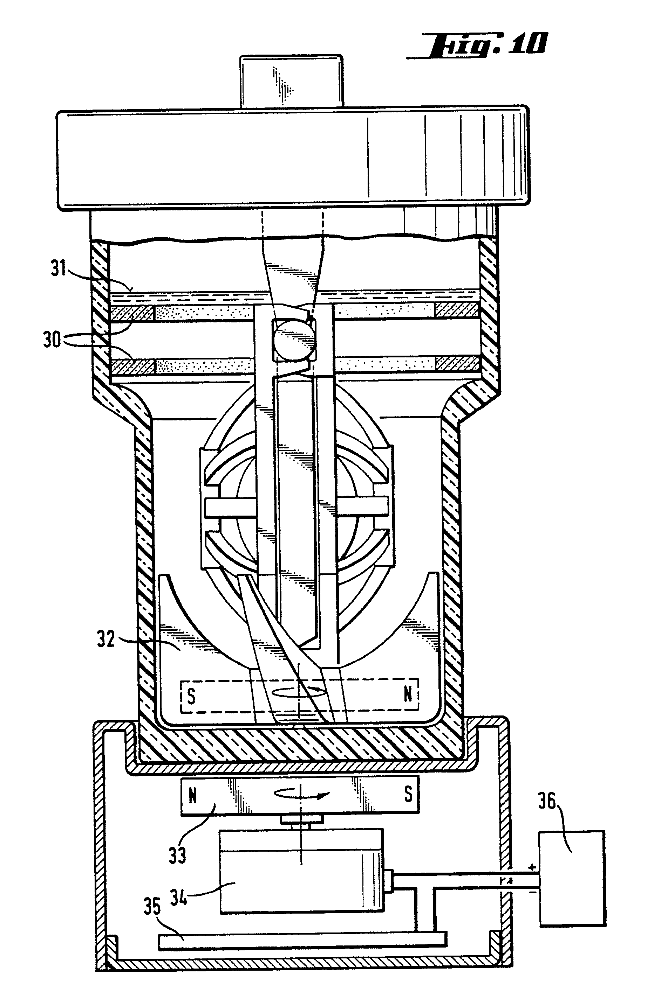

There is another means of controlling the decomposition rate of the hydrogen peroxide solution. This is by means of fixing the catalyst in a container at the top of the decomposition device so that it is contact with the hydrogen peroxide solution. Located at the bottom of the hydrogen peroxide decomposition device is a stirring means such as a stir bar for agitating the system such that fresh $H_2O_2$ is continually brought in contact with the catalyst. This is similar to the buoyancy mediated catalyst control system in that the contact between the catalyst and the reaction solution is controlled by means of the stirring action of the stir bar so that the reaction product solution is removed from the vicinity of the catalyst and hydrogen peroxide (the reaction solution) is continuously supplied to said catalyst as a result of the stirring action of the stir bar. This stirring, of course, is correlated such that the cumulative exposure is controlled so as to be at least 20 % peroxide·min over a period of no greater than 12 hours. Thus, according to this procedure the catalyst may be first immersed in the $H_2O_2$ solution without stirring and the stirring commenced after the $H_2O_2$ is decomposed in the vicinity of the catalyst and the reaction product solution thus-formed is subsequently removed and fresh $H_2O_2$ supplied by the subsequent stirring action. An example of such stir bar system is shown in Fig. 10. In Fig. 10 the catalyst (30) is below the solution level (31). A magnetic impeller (32) is driven by a drive magnet (33) which is connected to a motor (34). Further, a timer control (35) and the power supply (36) are shown. In all of these systems described, a conventional $H_2O_2$ decomposing device commonly used to disinfect contact lenses may be used, e.g. a commercially available AO Sept cup, but which has been modified to contain the stir bar features, hinge device and other decomposition means, etc., as described above.

### Other considerations

According to the general limitations of the present invention, the cumulative (percent peroxide)(min) exposure of the hydrogen peroxide from the point said contact occurs over a period of not greater than 12 hours is at least 20 % peroxide·min. However, in certain preferred instances, the period of exposure can be lessened to periods ranging from periods of no greater than 1.5 hours or periods ranging from periods no greater than 2 hours, 4 hours, 6 hours, 8 hours or no greater than 10 hours, depending upon the disinfectant objectives to be achieved, the degree of contamination of the device, etc. At the end of any of these time periods, the resulting solution should have a residual hydrogen peroxide content which is no greater than an ocularly acceptable amount, such that the contact lens can be inserted into the eye. Preferably, the hydrogen peroxide content should be no greater than about 400 ppm, preferably no greater than about 200 ppm, more preferably no greater than 100 ppm and in certain instances no greater than 75 ppm, still more preferably no greater than 60 ppm and ideally no greater than 30 ppm.

The hydrogen peroxide content at time zero should range from 0.5 to about 6 %, preferably 1 to about 4 %, more preferably about 2.5 to about 3.5 % and most preferably between about 3.0 to 3.5 %.

Further, as previously pointed out, the initial oxygen generation rate in conventional AO Sept system ranges from about 40 to 90 ml/min. These rates yield less than 10 %· minutes when correlated to the cumulative exposure. An initial rate of less than about 20 ml/min is required to yield 20 %·min according to the present invention. This is the cumulative exposure. The cumulative exposure over the full cycle until the time when the hydrogen peroxide reaches an ocularly acceptable level is referred to as the total exposure for the hydrogen peroxide system.

According to the present invention, the cumulative exposure of the material to be disinfected to the hydrogen peroxide between time zero and 15 minutes should be no more than 95 % of the total exposure. More preferably, the cumulative exposure to hydrogen peroxide between the time zero and 15 minutes should be preferably no more than 50 % of the total exposure.

Yet in another preferred limitation, the cumulative exposure to the hydrogen peroxide between time zero and 20 minutes later should be no more than 97 % of the total exposure and preferably no more than 65 % of the total exposure.

A cumulative exposure to hydrogen peroxide between time zero and 30 minutes later should be no more than 99 % of the total exposure and preferably no more than 85 % of the toal exposure. The cumulative exposure of the present invention should preferably be at least 20 % peroxide·min over a time ranging from about 15 to 30 minutes at the minimum in respect to the total exposure, preferably higher than 30 minutes, more preferably at least 60 minutes and most preferably at least about 1.5 hours at the minimum to a period of no greater than 12 hours.

As mentioned hereinbefore, the buoyancy mediated control of catalytic reaction is applicable also to other reactions than the catalytic decomposition of hydrogen peroxide. In such a method of buoyantly controlling a catalytic reaction it is a preferred embodiment that said catalyst is pure catalyst, catalyst coated substrate, or

EP 0 436 466 A2

catalyst impregnated substrate, and has a shape selected from a plate, a disc, a wafer, a sphere, a rod, a strip, a donut, an egg shape, a tear drop, a star, and gear shape ;
a density of from about 1.05 to about 1.30 ;
a surface area of about 0.2 cm² to about 5 cm² ; and
a weight/surface area ratio of about 0.85 to about 116.

Other than those specific embodiments disclosed in the present application, there are many other specific variations which will be apparent to those skilled in the art, once applicant's essential inventive concept is grasped.

## Claims

1. A method of disinfecting a hydrogen peroxide stable material comprising contacting said material with a hydrogen peroxide containing solution and a hydrogen peroxide decomposition means adapted to permit said material to have a cumulative (% peroxide) (min) exposure from the time said decomposition means contacts said peroxide containing solution, time zero, over a period of no greater than 12 hours of at least 20%peroxide · min.

2. The method of claim 1 wherein said material is a polymeric plastic.

3. The method of claim 1 wherein said material is an ophthalmic device.

4. The method of claim 1 wherein said material is a contact lens.

5. A method of decomposing a hydrogen peroxide solution comprising contacting said solution with a hydrogen peroxide decomposition means adapted to permit a material placed in said solution to have a cumulative (% peroxide) (min) exposure to hydrogen peroxide from the point said contacting occurs over a period of not greater than 12 hours of at least 20 % peroxide · min.

6. The method of claim 1 wherein said period is no greater than about 1.5 hours.

7. The method of claim 1 wherein said material is ocularly insertable and the resulting solution has a residual hydrogen peroxide content at the conclusion of said period which is no greater than an ocularly acceptable amount.

8. The method of claim 7 wherein the residual hydrogen peroxide content is no greater than about 30 ppm.

9. The method of claim 1 wherein said hydrogen peroxide containing solution has a hydrogen peroxide content at time zero of from about 0.5 to about 6 %.

10. The method of claim 9 wherein said hydrogen peroxide content at time zero is from about 3.0 to about 3.5 %.

11. The method of claim 1 wherein said material has a cumulative (% peroxide) (min) exposure between time zero and 15 minutes later of no more than 95 % of the total exposure.

12. The method of claim 1 wherein said material has a cumulative (% peroxide) (min) exposure between time zero and 20 minutes later of no more than 97 % of the total exposure.

13. The method of claim 1 wherein said material has a cumulative (% peroxide) (min) exposure between time zero and 30 minutes later of no more than 99 % of the total exposure.

14. The method of claim 1 wherein said decomposition means is a catalyst.

15. The method of claim 1 wherein said decomposition means is reactive with hydrogen peroxide.

16. A method of decomposing a hydrogen peroxide solution according to claim 5 by buoyantly controlling the catalytic reaction comprising contacting said hydrogen peroxide solution and a particulate catalyst capable of converting said hydrogen peroxide solution into reaction products, said particulate catalyst having a density such that it sinks into the reactant medium but generates a positive buoyant force which adheres for a time to said particle driving said particle toward the reactant solution surface or total solution surface, the reaction product thereof being as dense or less dense than said reactant medium.

17. The method of claim 16 wherein said catalyst is selected from transition metals and compounds thereof and enzymes.

18. The method of claim 17 wherein said enzymes are selected from peroxidase and catalase.

19. The method of claim 17 wherein said transition metal is selected from platinum, palladium, iron, manganese, and titanium, and said transition metal compounds are the oxides of said transition metals.

20. The method of claim 17 wherein said catalyst is selected from platinum and catalase.

21. The method of claim 16 wherein said catalytic reaction is the catalytic decomposition of aqueous hydrogen peroxide and said catalyst is present as a shaped substrate having said catalyst coated thereon or impregnated therewith, said substrate impregnated with catalyst having sufficient porosity to aqueous hydrogen peroxide to permit said catalytic reaction to occur and allow escape of reaction product from the internal portions of said substrate.

22. The method of claim 21 wherein said substrate is a polymer of natural or synthetic origin resistant to the chemical effects of reactant, reaction product and catalyst.

23. The method of claim 22 wherein said substrate is a synthetic polymer selected from polycarbonate alone or in admixture with acrylate butadiene styrene or with noryl.

24. The method of claim 23 wherein said substrate is polycarbonate.

25. The method of claim 21 wherein said substrate is coated with said catalyst.

26. The method of claim 16 wherein said catalyst is pure catalyst, catalyst coated substrate, or catalyst impregnated substrate, and has a shape selected from a plate, a disc, a wafer, a sphere, a rod, a strip, a donut, an egg shape, a tear drop, a star, and gear shape ;
a density of from about 1.05 to about 1.30 ;
a surface area of about 0.2 $cm^2$ to about 5 $cm^2$ ; and
a weight/surface area ratio of about 0.85 to about 116.

27. The method of claim 26 wherein the catalyst is present as a coating on a substrate, and the catalyst is platinum or catalase.

28. The method of claim 27 wherein said catalyst coated substrate has a sphere shape.

29. The method of claim 27 wherein said catalyst coated substrate has a density of about 1.12 to about 1.18.

30. The method of claim 27 wherein said catalyst coated substrate has a surface area of about 0.3 $cm^2$ to about 3.2 $cm^2$.

31. The method of claim 27 wherein said catalyst coated substrate has a weight to surface area ratio of about 2.95 to about 11.3.

32. The method of claim 16 wherein said hydrogen peroxide content remain present in a disinfectingly effective concentration in the vicinity of an article to be disinfected therein for at least 15 minutes after said catalytic reaction has begun.

33. The method of claim 32 wherein said article to be disinfected therein is a contact lens.

34. A method according to claim 5 of decomposing a hydrogen peroxide solution by buoyant control comprising contacting said solution with freely moving catalytic particles which upon making said contact generate sufficiently adherent oxygen bubbles to give said particles a buoyant force whereby said particles are lifted toward the surface of said solution until said oxygen bubbles are lost whereupon said particle sinks through said solution until sufficient buoyant force is generated by further catalytic decomposition and generation of further adherent oxygen bubbles such that a non-homogeneous decomposition takes place beginning near the top of the peroxide solution and ending at the bottom thereof.

35. The method of claim 34 wherein said peroxide solution starts at a concentration of about 3.0 to about 3.5 % hydrogen peroxide.

36. The method of claim 34 wherein said hydrogen peroxide decomposition is substantially complete such that said solution is ocularly compatible within a first time period of about 8 hours.

37. The method of claim 34 wherein said hydrogen peroxide concentration remains in excess of 1 % at the bottom of the solution for a second time period of at least 15 minutes.

38. A hydrogen peroxide decomposition means for use in the method of claim 1 comprising buoyant, free floating particles of a hydrogen peroxide decomposition agent, alone or coated on or contained within an inert, hydrogen-peroxide-decomposition agent carrier.

39. A hydrogen peroxide decomposition means for use in the method of claim 1 comprising a shaped catalyst, alone or in a carrier therefor, movably attached to a portion of a container in which said hydrogen peroxide decomposition is carried out.

40. The decomposition means of claim 39 wherein said shaped catalyst is movably attached to a wall of said container.

41. The decomposition means of claim 39 wherein said container comprises at least one holding portion to hold the material to be disinfected, said catalyst being movably attached to at least one of said holding portions.

42. A device for the decomposition of hydrogen peroxide in the presence of a material being disinfected therein, said device having material for disinfection and hydrogen peroxide solution containment areas such that said solution is in contact with said material, a decomposition means containment area such that when said decomposition means is present it is in contact with said solution, a device closure means for closing said device, and a pressure releasing means for releasing the pressure resulting from the hydrogen peroxide decomposition reaction, said decomposition means being a member selected from the group consisting of

(a) a catalyst fixed to a position which is at the bottom of said solution, said catalyst being prepoisoned to partially deactivate the active sites of the catalyst to slow down the decomposition rate of the hydrogen peroxide solution,

(b) a catalyst flexibly attached to a portion of said device so as to move out of and into said solution during said decomposition process,

(c) a catalyst enclosed within a container located within said hydrogen peroxide decomposing device, said container having one or more orifices therethrough so as to restrict the flow rate of oxygen from inside the container to the exterior of the container,

(d) a catalyst free floaring in said solution with a density to drive it into unreacted solution areas and sufficiently light to be driven away from said unreacted solution areas by buoyant forces resulting from oxygen bubbles adherent thereto, and

(e) a catalyst located in a position at the top of the device and which is in contact with the hydrogen peroxide solution, whereby the decomposition of the hydrogen peroxide is controlled by a stirring means located at the bottom of the hydrogen peroxide decomposition device which stirring means functions by agitating the hydrogen peroxide liquid solution such as to remove the reaction product formed from the vicinity of the catalyst and bring the unreacted hydrogen peroxide in fresh contact with the catalyst,

said decomposition means adapted to decompose hydrogen peroxide solution down to at least ocularly acceptable concentrations within no more than 12 hours and expose said material to a cumulative hydrogen peroxide amount of at least 20 % peroxide·min.

*Fig. 1* [PRIOR ART]

*Fig. 2*

Fig. 3

EP 0 436 466 A2

Fig. 4

Fig. 5

Fig: 6

*Fig. 7*

*Fig. 8*

EP 0 436 466 A2

_Fig. 9_

EP 0 436 466 A2

**Fig. 10**